# EUROPEAN PATENT SPECIFICATION

(11) **EP 1 985 647 B1**
(45) Date of publication and mention of the grant of the patent: **16.03.2011**
(21) Application number: 06807859.1
(22) Date of filing: 17.07.2006
(51) Int. Cl.: C08H 1/06, A61K 8/98, D01F 4/00

(54) **METHOD OF OBTAINING KERATIN MICROFIBRES FROM LIVESTOCK WASTE**
VERFAHREN ZUM ERHALT VON KERATINMIKROFASERN AUS VIEHABFÄLLEN
PROCEDE D'OBTENTION DE MICROFIBRES DE KERATINE A PARTIR DE RESIDUS DE GIBIER

(30) Priority: 23.08.2005 ES 200502083
(43) Date of publication of application: 29.10.2008
(73) Proprietor: Asesoramiento Tecnico Medioambiental Energy, S.L., 41010 Sevilla (ES); Florido Rodriquez, José Luis, 41010 Seville (ES)
(72) Inventor: FLORIDO RODRIGUEZ, José Luis, E-41010 Sevilla (ES)
(74) Representative: Gonzalez-Mogena Gonzalez, Inigo
(86) International application number: PCT/ES2006/000410
(87) International publication number: WO 2007/023199

(56) References cited:
- EP-A2- 0 499 260
- WO-A-03/018673
- WO-A1-03/006531
- US-A- 3 464 825
- US-B1- 6 274 155

## Description

### SUBJECT OF THE INVENTION

This invention refers to a method of obtaining keratin microfibres from livestock waste, which provides essential features of novelty and notable advantages over the methods currently known and used for this purpose in the current state of the art.

More specifically, the invention proposes the development of a method through which it is possible to obtain keratin microfibres by way of cold hydrolysis from the hydrolysed protein of agricultural waste such as feathers, hair, hide, hooves or horns.

The field of application of the invention is within the industrial sector involved with the treatment of such agricultural waste, especially for obtaining keratin for various industrial uses.

### BACKGROUND OF THE INVENTION

It is understood that in the state of the art there exists a large amount of waste produced by the livestock industry that contains, in varying proportions, the keratin protein, both in its alpha and beta forms. As is known, keratin is an albuminoidal substance, very rich in sulphur, which forms the fundamental part of the most outer layers of the epidermis of vertebrates and the organs derived from this membrane, such as feathers, hide, horns, nails or hooves; in particular, keratin is the substance responsible for the toughness and resistance of horns and nails.

Furthermore, although the use of these keratin sources is a well-known industrial process, most methods of obtaining keratin from agricultural waste are based on very aggressive thermal or chemical treatment procedures. For example, one may cite documents ... ...., all of which are included here by way of reference.

As a result of the aggressive nature of these treatment procedures, the protein that is obtained is considerably denatured and loses most of the properties that make keratin a protein of interest for industrial use.

In 1981, Yoshioka Issei proposed a method based on cold hydrolysis for cosmetic uses, using an alkali and a metal sulphide to break the disulphuric bonds of the amino acids and obtain hydrolysis with the protein in a good state.

Later work undertaken by Schrooyen PM et al showed that protein obtained by cold hydrolysis is reactive and can be interwoven to obtain new materials.

The invention now proposed here is aimed in both directions, as it consists of a method that allows keratin to be obtained through hydrolysis of protein from agricultural waste obtained by non-thermal hydrolysis. The process, carried out in cold conditions, has two variants. The first of these allows keratin microfibres to be obtained with the keratin retaining all of its properties, and it is therefore applicable for use in several sectors of the state of the art, such as the textile sector, as a means of reinforcement in the production of industrial polymers such as polyethylene or polypropylene, the production of paste for paper or cardboard and the production of biomaterials or biomedical materials, among others. The second variant allows for soluble keratin to be obtained for use in the cosmetic and food industries.

### DESCRIPTION OF THE PREFERRED EMBODIMENT

The remainder of the description will explain the method of obtaining keratin in cold conditions from livestock waste proposed by the invention, with specific reference to the case of feathers. However, this choice should be considered purely illustrative of the process, for the purpose of making this description easier to understand, the description being applicable, in the same way, to any of the other livestock waste considered here (hair, hide, hoof, horns).

In accordance with the invention, the method envisages the implementation of a series of consecutive stages for obtaining insoluble keratin microfibres. These stages are set out below:

### Obtaining insoluble keratin microfibres

a) Shredding and hydrolysis: In this first stage the feathers from livestock residue are shredded with the aid of an industrial shredder, and after shredding, sodium sulphide and water are added to achieve hydrolysis, which will last for approximately one day, during which time it will be agitated. The minimum amount of water is determined by the height of the propeller of the pneumatic agitator being used.
b) Oxidisation and adjustment of pH to an acid value: After the hydrolysis of the previous stage, the liquid is emptied from the tank from the base. The liquid is transferred to an oxidisation and pH adjustment tank (diameter, height), to which H²O², ozone or other product with an oxidising effect, is added, and the contents of the tank are agitated for around 50 minutes with a pneumatic agitator; the pH is adjusted by adding sulphuric acid until pH acid is achieved (between 4 and 5).
c) 1st Decantation: The pH acid liquid from the previous stage is left to decant in the same agitation tank for around three hours. Next, suction is applied to the liquid with the aid of a peristaltic pump, and the solid is collected from the upper part of the tank.
d) Adjustment to pH 7, and 1^{st} wash: The solid collected in the previous 1^{st} decantation stage is dissolved in water and its pH is then adjusted to a value of pH 7 by adding NaOH. Next, it is left under agitation for 30 minutes in order to wash it.
e) 2^{nd} decantation: After the agitation period of the previous stage has finished, the solution is left to decant for around three hours before the liquid is removed with the aid of a pump.
f) 2^{nd} wash: Water is added once again and the contents are agitated for around another 30 minutes.
g) 3^{rd} decantation: After another decantation for a period of around three hours, the liquid is separated from the solid in the same way as in the previous decantation stages, and the solid obtained is submitted to a polymerisation stage.
h) Polymerisation: For the polymerisation process, after the hydrolysed product has been obtained at pH 7, it is desalinised through ultrafiltration and then adipic acid (between 0.1 and 10%) is added to it before reprecipitation to pH acid. The aim of this phase is to achieve greater polymerisation of the product and larger fibres. Addition and re-polymerisation is carried out by slow (between 1 and 24 hours) heating (to between 30 and 80 degrees Celsius).

### EXAMPLES OF EMBODIMENT

### Example 1

- Washing of feathers: 1 Kg of feathers are weighed and put into a metal basket (diameter: 39 cm, height: 36.5 cm) positioned inside the washing tank. The feather washing tank is made of PVC (external diameter: 56.6 cm, internal diameter: 55 cm, height 50 cm), and has a grille 8.5 cm from the base positioned to stop feathers passing through to the bottom, where the recirculation system is. The liquid is propelled by a centrifugal pump that circulates the liquid to the upper central part of the washing tank, which ensures that all of the water comes into contact with the feathers. The duration of the washing stage is one day, and after it is finished, the basket with the feathers is removed, to proceed to the draining stage, and the washing liquid is emptied with the aid of a pump (this liquid is discarded).
- Draining: The feathers within the basket are drained and transferred to a second tank in which the shredding and hydrolysis of the feathers will be undertaken.
- Shredding and hydrolysis: The shredding and hydrolysis tank is made of PVC (external diameter: 51 cm, internal diameter: 50 cm, height 50 cm) and also has a grille positioned 6.5 cm from the base. The shredding tank has a lid in which a pneumatic agitator is installed, and also an emptying valve in the central part of the base. In this stage, the feathers from the previous stage are shredded with an industrial mixer and a stated amount of sodium sulphide (preferably around 100 grams) and a minimum amount of 30 litres of water is added in order to achieve hydrolysis, for approximately a day, during which time agitation is maintained by the pneumatic agitation equipment. The minimum amount of water depends on the height of the propeller of the pneumatic agitator, as the agitation would not be adequate with a lower amount of water.
- Addition of H²O² and adjustment to pH 4.8: After hydrolysis, the liquid is emptied from the tank from the base. The liquid is transferred to another tank for addition of H²O²² and adjustment of pH (diameter, height) and 160 ml of H²O² is added and the tank is agitated for around 50 minutes with a pneumatic agitator, and pH is adjusted by adding sulphuric acid (20%).
- 1^{st} Decantation: The liquid at pH 4.8 is left to decant in the same agitation tank for around three hours. Next, suction is applied to the liquid with the aid of a peristaltic pump, and the solid is collected from the upper part.
- Adjustment to pH 7, and 1^{st} wash: The solid collected from the decantation is dissolved in 6 litres of water in a 10 litre beaker, and its pH is adjusted to a value of pH 7 by adding NaOH. Next, it is left under agitation for around 30 minutes (magnetic agitator) in order to wash it.
- 2^{nd} Decantation: Agitation is stopped and decantation is allowed to take place for around three hours in the same beaker, the liquid then being taken out with the use of a peristaltic pump.
- 2^{nd} Wash: Another six litres of water are added again and the contents of the beaker are agitated for a further 30 minutes.
- 3^{rd} Decantation: After around three hours of decantation, the liquid is separated from the solid in the same way as in the previous decantation stages and the obtained solid is transferred to the spraying stage.
- Polymerisation: The product obtained is the protein in the form of fibres, but when making fibre for plastics extrusion, the more developed the protein fibre is, the better the properties (elasticity, resistance...) granted to the obtained fibre are. Therefore, although a fibrous product is obtained naturally, a series of polymerisation processes are introduced in order to achieve a consistent primary polymerisation. The resultant hydrolysed product is treated with adipic acid at 1% before reprecipitation to pH 4.8. The aim of this stage is to achieve greater polymerisation of the product and larger fibres. Addition and re-polymerisation is carried out with the slow (24 hours) addition of heat (37 degrees Celsius).

The results are observed through a Scanning Electron Microscope (SEM), which enables the surface of the materials to be seen and the structure of these surfaces to be observed on a micrometric scale. At the same time it allows the chemical composition of the surface to be determined by EDS (Energy Dispersive Spectroscopy).

It is not considered necessary to go into further detail in this description for an expert to be able to understand the scope of the present invention and the advantages entailed. However, it must understood that the invention has been described in relation to its preferred embodiment, and it is therefore subject to alterations and variations both in the stages of the process and in the conditions under which the stages take place, without these changes being considered beyond the scope of the invention, defined in the claims that follow.

## Claims

1. Method of obtaining insoluble keratin microfibres from livestock waste, in particular from livestock waste such as feathers, hair, hide, hooves or horns, the keratin fibres obtained from which retain the properties of the natural fibres, which is **characterised** because it consists of the following stages:
a) shredding and cold hydrolysis of the livestock waste, with the aid of an industrial shredder and the subsequent addition of sodium sulphide and water in order to achieve hydrolysis, during a period of approximately one day, under continual agitation with a pneumatic agitator;
b) oxidisation and adjustment of pH to an acid value, following the previous hydrolysis, for which purpose the liquid is emptied from the treatment tank, from the base of the tank, and transferred to an oxidisation and pH adjustment tank, where H₂O², ozone or other product with an oxidising effect is added, and the contents of the tank are agitated for around 50 minutes with a pneumatic agitator, pH being adjusted with the addition of sulphuric acid until a pH acid value is achieved (between 4 and 5);
c) decantation of the liquid at pH acid from the previous stage, carried out in the same agitation tank for around three hours, after which a suction phase is carried out on the liquid with the aid of a peristaltic pump, the solid then being collected from the upper part of the tank;
d) adjustment to pH 7, and 1^{st} wash, dissolving the solid collected in the previous decantation stage in water, and then proceeding to adjust pH to a value of pH 7 with the addition of NaOH, subjecting all of that to agitation for approximately 30 minutes, in order to wash it;
e) another decantation after the agitation period of the previous stage, for a period of around three hours, with subsequent removal of the liquid with the aid of a pump;
f) 2^{nd} washing stage, for which water is added again and the contents agitated for around another 30 minutes;
g) additional decantation stage for a period of around three hours, with the aim of separating the liquid from the solid in the same way as in the previous decantation stages, the solid being submitted to a final stage of polymerisation, and
h) polymerisation stage, in which, after the hydrolysed product has been obtained at pH 7, the product is desalinised by ultrafiltration with subsequent addition of adipic acid (between 0.1 and 10%) before reprecipitation to pH acid. The aim of this phase is to achieve greater polymerisation of the product and larger fibres, the addition and re-polymerisation being carried out through the slow (between 1 and 24 hours) addition of heat (between 30 and 80 degrees Celsius).

2. Insoluble keratin microfibres obtained by the cold hydrolysis method of claim 1, above, originating from livestock waste such as feathers, hair, hide, hooves or horns, with better properties of resistance and elasticity.

3. Use of the keratin microfibres from claim 2 in textile applications.

4. Use of the keratin microfibres from claim 2 in the reinforcement of industrial polymers (polyethylene and polypropylene).

5. Use of the keratin microfibres from claim 2 in the production of paste for paper or cardboard.

6. Use of the keratin microfibres from claim 2 in the manufacture of biomaterials and biomedical materials.

## Patentansprüche

1. verfahren für die Gewinnung von Mikrofasern aus unlöslichem Keratin ausgehend von Resten aus der Viehhaltung, insbesondere von Resten aus der Viehhaltung wie Federn, Haar, Haut, Klauen oder Hörnern, deren keratinhaltigen Fasern nach der Gewinnung die Eigenschaften der natürlichen Fasern beibehalten, **dadurch gekennzeichnet, dass** es folgende Schritte aufweiset:
a) Zerkleinerung und Hydrolyse der Reste aus der Viehhaltung in kaltem Zustand, mit Hilfe einer industriellen Zerkleinerungsmschine und anschließender Zugabe von Natziumsulfid und Wasser zur Durchführung der Hydrolyse über den Zeitraum von ungefähr einem Tag, unter ständigem Rühren mit einem pneumatisch betriebenen Rührwerk;
b) nach Beendigung der vorherigen Hydrolyse, Oxidierung und Einstellung des pH-Werts auf einen sauren Wert, wofür die Flüssigkeit am unteren Teil aus dem Behandlungstank entleert wird, wonach die Flüssigkeit zu einem Tank für die Oxidierung und die Einstellung des pH-Werts geleitet wird, in dem H₂O₂, Ozon oder ein anderes Oxidierungsmittel zugegeben wird, unter Rühren des Tankinhalts über einen Zeitraum von ungefähr 50 Minuten mit einem pneumatisch betriebenen Rührwerk, wobei die Einstellung des pH-Werts durch Zugabe von Schwefelsäure stattfindet, bis ein saurer pH-Wert (zwischen 4 und 5 erreicht wird);
c) Dekantierung der Flüssigkeit mit dem sauren pH-Wert aus dem vorhergehende Schritt, was im gleichen Rührtank über einen Zeitraum von ungefähr drei Stunden durchgeführt wird, wonach die Flüssigkeit mit Hilfe einer Peristaltikpumpe abgesaugt wird und der Feststoff am unteren Teil des Tanks gesammelt wird;
d) Einstellung des pH-Werts auf 7, und 1. Spülung, wobei der im vorhergehenden Schritt der Dekantierung gesammelte Feststoff in Wasser gelöst wird und im Anschluss der pH-Wert durch Zugabe von NaOH auf 7 eingestellt wird, wobei alles über einen Zeitraum von ungefähr 30 Minuten gerührt wird, um so die Spülung durchzuführen;
e) neuer Schritt der Dekantierung nach Ablauf der Rührzeit des vorhergehenden Schritts für einen Zeitraum von ungefähr drei Stunden, wonach die Flüssigkeit mit Hilfe einer Pumpe abgezogen wird;
f) 2. Spülschritt, wofür erneut Wasser zugegeben wird und der Inhalt über einen Zeitraum von ungefähr weiteren 30 Minuten gerührt wird;
g) zusätzlicher Schritt der Dekantierung über einen Zeitraum von ungefähr drei Stunden, um die Flüssigkeit vom Feststoff zu trennen, was auf die gleiche Weise wie bei den vorhergehenden Dekantierungen stattfindet, wonach der gewonnene Feststoff einem letzten Schritt in Form einer Polymerisation unterworfen wird, und
h) Schritt der Polymerisation, der darin besteht, dass nach Gewinnung des hydrolysierten Produkts mit einem pH-Wert 7 durch Ultrafiltration entsalzt und anschließend Adipinsäure (0,1 bis 10%ig) hinzugegeben wird, bevor durch erneute Ausfällung wieder ein saurer pH-Wert eingestellt wird. Das Ziel dieser Verfahrensstufe besteht darin, eine höhere Polymerisation des Produkts sowie größer Fasern zu erreichen, wobei die Zugabe und erneute Polymerisation langsam (1 bis 24 Stunden lang) unter Wärmezuführung (30 bis 80°C) durchgeführt wird.

2. Unlösliche Mikrofasern aus Keratin, gewonnen durch ein Hydrolyseverfahren in kaltem Zustand nach dem vorhergehenden Anspruch 1, ausgehend von Resten aus der Viehhaltung, wie Federn, Haar, Haut, Klauen oder Hörnern, mit verbesserten Figenschaften hinsichtlich der Festigkeit und Elastizität.

3. Benutzung der Mikrofasern aus Keratin nach Anspruch 2 für Textilanwendungen.

4. Benutzung der Mikrofasern aus Keratin nach Anspruch 2 bei der Verstärkung industrieller Polymere (Polyethylene und Polypropylone).

5. Benutzung der Mikrofasern aus Keratin nach Anspruch 2 bei der Herstellung von Papier- oder Kartonbrei.

6. Benutzung der Mikrofasern aus Keratin nach Anspruch 2 bei der Herstellung von Biomaterialien und biomedizinischen Materialien.

## Revendications

1. Procédé destiné à l'obtention de microfibres de kératine insoluble à partir de déchets d'élevage, notamment à partir de déchets d'élevage comme les plumes, les poils, la peau, les onglons ou les cornes, dont les fibres kératiniques obtenues conservent les propriétés des fibres naturelles ; un procédé qui se **caractérise par** les étapes suivantes :
a) broyage et hydrolyse à froid des déchets d'élevage, à l'aide d'un broyeur industriel et de l'addition postérieure de sulfure de sodium et d'eau pour réaliser l'hydrolyse, pendant approximativement un jour, sous agitation continue avec un agitateur pneumatique ;
b) oxydation et ajustement du pH à une valeur acide, après avoir réalisé l'hydrolyse antérieure ; à cette fin on effectue la vidange du liquide à partir du réservoir de traitement, par la base de celui-ci, le liquide étant conduit vers un réservoir d'oxydation et d'ajustement du pH, où a lieu l'addition de H₂O₂, d'ozone ou autre substance oxydante, avec agitation du contenu du réservoir pendant approximativement 50 minutes à l'aide d'un agitateur pneumatique, l'ajustement du pH s'effectuant avec l'addition d'acide sulfurique jusqu'à obtenir une valeur de pH acide (entre 4 et 5);
c) décantation du liquide à pH acide de l'étape antérieure, effectuée dans le même réservoir d'agitation pendant approximativement trois heures, puis procéder à une phase d'aspiration du liquide à l'aide d'une pompe péristaltique, et collecte du solide par la partie inférieure du réservoir ;
d) ajustement à pH 7, et 1^{er} lavage, en dissolvant dans de l'eau le solide collecté durant l'étape de décantation antérieure, et procéder ensuite à l'ajustement du pH 7 avec l'addition de NaOH, en laissant le tout sous agitation pendant approximativement 30 minutes, pour effectuer le lavage ;
e) nouvelle étape de décantation après avoir réalisé l'étape d'agitation antérieure, pendant approximativement trois heures, puis retirer le liquide à l'aide d'une pompe ;
f) 2^{ème} étape de lavage ; à cet effet, ajouter de nouveau de l'eau et agiter le contenu pendant approximativement 30 autres minutes ;
g) étape de décantation additionnelle pendant approximativement trois heures, afin de séparer le liquide du solide comme lors des décantations antérieures, et en soumettant le liquide obtenu à une étape finale de polymérisation, puis
h) étape de polymérisation durant laquelle, après l'obtention du produit hydrolysé à pH 7, on le déminéralise par ultrafiltration et postérieurement on lui ajoute de l'acide adipique (entre 0,1 et 10 %) avant la reprécipitation à pH acide. L'objectif de cette phase est d'obtenir une polymérisation plus importante du produit et des fibres plus grandes en réalisant l'addition et la repolymérisation avec l'addition de chaleur (entre 30 et 80°C), lentement (entre 1 et 24 h).

2. Des microfibres de kératine insolubles obtenues à travers le procédé d'hydrolyse à froid de la revendication 1 qui précède, issues de déchets d'élevage comme les plumes, les poils, la peau, les onglons ou les cornes, avec des propriétés de résistance et d'élasticité améliorées.

3. Utilisation des microfibres de kératine de la revendication 2, pour des applications textiles.

4. Utilisation des microfibres de kératine de la revendication 2, dans l'effort des polymères industriels (polyéthylènes et polypropylénes).

5. Utilisation des microfibres de kératine de la revendication 2, dans la production de pâte à papier ou de carton.

6. Utilisation des microfibres de kératine de la revendication 2, dans la fabrication de biomatériaux et de matériaux biomédicaux.
